# EUROPEAN PATENT APPLICATION

(11) **EP 4 049 688 A1**
(43) Date of publication of application: **31.08.2022**
(21) Application number: 21159003.9
(22) Date of filing: 24.02.2021
(51) Int. Cl.: A61L 24/08, A61L 26/00

(54) **SELF-SUPPORTING VISCIN FILMS AND SCAFFOLDS, USES THEREOF AND METHODS FOR PREPARING THE SAME**

(71) Applicant: Max-Planck-Gesellschaft zur Förderung der Wissenschaften e.V., 80539 München (DE)
(72) Inventor: HARRINGTON, Matthew, Montreal, QC H4A 1J2 (CA); FRATZL, Peter, 14195 Berlin (DE); HORBELT, Nils, 12435 Berlin (DE)
(74) Representative: v. Bezold & Partner Patentanwälte - PartG mbB

(57) **Abstract**

The invention relates in a first main aspect to self-supporting mistletoe viscin films comprising a 2-dimensional multi-axial oriented array of viscin cellulose filaments within a humidity-responsive matrix and methods for preparing the same. A further aspect relates to 2D and 3D mistletoe viscin scaffolds and methods for preparing the same.

Another main aspect of the invention relates to the use of mechanically isolated mistletoe viscin or of said self-supporting mistletoe viscin films for joining or binding together a plurality of materials with diverse surface characteristics and to an adhesive comprising such viscin films and/or 2D or 3D viscin scaffolds.

In more specific embodiments, the invention relates to a wound sealant and coating composition or to a medical kit comprising mechanically isolated mistletoe viscin in the hydrated/wet state and a plant oil, or a dried viscin film for use, after rehydration under humid conditions, as a wound sealant.

## Description

### Background of the invention

In view of the increasing environmental concerns worldwide, there is currently a strong demand for sustainable alternatives for polymeric materials and adhesives that do not rely on petroleum-based feed-stocks. Consequently, there is growing interest in establishing and utilizing plant-based materials, e.g. sources of cellulose, for a variety of applications.

More specifically, there is also broad need in technical and biomedical disciplines for new and versatile adhesives with favorable characteristics which preferably are also eco-friendly and biocompatible.

The berries of the mistletoe plant (Viscum album) contain a cellulose-based adhesive substance surrounding the seed, which is commonly known as viscin. A natural glue made from such mistletoe berries has been used in the past as a birdlime to capture birds. Partly purified viscin from various sources has also been described as a component of wound dressings and plasters (Riehl, Deutsche Medicinische Wochenschrift, 41, October 11, 1900).

N. Horbelt, M. Eder, L. Bertinetti, P. Fratzl and M. Harrington (Biomacromolecules 2019, 20, 3094-3103) disclose that the mucilaginous viscin tissue can be processed under ambient conditions into stiff cellulosic fibers.

However, the prior art neither discloses nor suggests a method for preparing self-supported and oriented viscin films and scaffolds from isolated viscin tissue and the use thereof for various adhesive applications. As determined by the present inventors, such films exhibit locally oriented cellulose microfibrils, which enhance mechanical integrity of the films, while scaffold architecture can be controlled at multiple length scales for the specific needs, providing distinct advantages and improvements over previously described systems.

Also, this prior art does neither disclose or suggest the excellent adhesion properties of viscin with respect to a very large variety of quite different substrates nor several other favorable properties of viscin which renders this material especially suited for various novel adhesive applications.

Therefore, a main object underlying the present invention is the provision of improved adhesive materials which overcome or considerably alleviate the drawbacks of the prior art in that they are widely applicable, in particular suitable for binding and gluing a wide plurality of materials with diverse surface characteristics, including plastics, metals, glass, ceramics but also living animal tissue and skin, are eco-friendly and biocompatible and can be a manufactured in a relatively simple and cost-efficient manner.

This main object is achieved according to the present invention by providing the self-supporting viscin film according to claim 1 and the method for preparing the same according to claim 4. Additional aspects and preferred embodiments of the invention are the subject of further claims.

### Description of the invention

The self-supporting 2D mistletoe viscin film according to the present invention comprises a 2-dimensional multi-axial oriented array of viscin cellulose filaments within a matrix.

More specifically, the self-supporting 2D mistletoe viscin film according to the invention has locally oriented cellulose filaments embedded within a humidity-responsive matrix and exhibits adhesive properties in the hydrated/wet state and is non-adherent and optically transparent in the dehydrated/dry state.

The oriented cellulose filaments held together by the humidity-responsive matrix provide stiff, yet flexible material properties to the films. Upon drying, the films are extremely stable but capable to revert to the hydrated and adhesive state under humid conditions.

A freshly formed (or rehydrated) adhesive viscin film can be applied onto a wide range of hydrophobic and hydrophilic surfaces (including mammalian skin and cartilage) and readily adapts to various surface shapes and surface topographies, e.g. curved or flat, rough or smooth surfaces.

The term "viscin", as used herein, refers to the mucilaginous tissue surrounding the pseudoseeds of mistletoe plants, the tissue comprising viscin cells, which cells exhibit a characteristic cell wall structure of coiled cellulose fibrils and are organized in clusters or bundles in a natural humidity-responsive adhesive matrix.

The cell walls of pristine viscin cells consist of cellulose fibrils with an unusual orientation - perpendicular to the cell long axis - which are embedded into a non-cellulosic matrix. These cells can be mechanically drawn into micron-sized filaments which co-align in the drawing direction to form macroscopic viscin fibers or orient themselves along the directions of multiaxial tensile loads to form 2D viscin films.

Principally, the mistletoe viscin used in the present invention may be isolated from a variety of mistletoe plants, provided that the corresponding viscin cells exhibit the above described characteristic cell wall structure of coiled cellulose fibrils and are organized in clusters or bundles in a natural humidity-responsive adhesive matrix.

More specifically, the viscin used in the present invention is a mistletoe viscin derived from pseudoberries of mistletoe plants selected from the group comprising the European mistletoe, i.e. *Viscum album* L., and related species or subspecies, in particular Viscum minimum, Viscum cruciatum, Viscum album L. coloratum, Viscum album var. rubro-aurantiacum Makino, Viscum album L. var. lutescens Makino, and Phthirusa pyrifolia.

The present invention also provides a method for the mechanical isolation of viscin which comprises at least the following steps:
a) opening the pseudoberries of mistletoe plants by mechanical means, e.g. applying compression forces to the pseudoberries or cutting the pseudoberries, b) extracting the seeds, c) separating the viscin tissue attached to the seeds and the pseudoberry skins from said seeds and pseudoberry skins, and d) optionally further processing or purifying of the viscin tissue obtained in step c).

It is crucial to isolate the viscin tissue by purely mechanical means, since it has been found that the use of chemical extraction or pulping steps results in a deterioration or even complete loss of essential properties of the viscin tissue obtained therewith.

Another aspect of the present invention relates to a method for preparing a self-supporting 2D viscin film as characterized above which comprises at least the following steps:
a) providing mechanically isolated viscin tissue, e.g. by the method as described above,
b) applying a multi-axial tensile load onto said viscin tissue, resulting in the formation of a viscin film comprising a 2-dimensional array of viscin cellulose filaments oriented locally along the direction of the tensile load during formation,
c) drying the resulting film, preferably under ambient conditions, in particular air drying at a temperature in the range from 15°C to 30°C, preferably from 20°C to 25°C and at a relative humidity RH of less than 40 %, but preferably lower.
d) optionally further processing after rehydration.

In one specific embodiment, step b) comprises i) forming hydrated viscin tissue into one or more self-supporting strands and anchoring the same in a predetermined distance at two elevated points of a substrate, e.g. onto the edges of a Petri dish, by means of the natural adhesion of the viscin and without additional contact with the substrate, ii) subsequently drawing the still hydrated viscin strand along at least one other axis forming a 2D film with at least three anchor points.

Extensive studies of the present inventors have revealed that the viscin fibers and films disclosed herein exhibit several surprising and very favorable properties, in particular:
- dried fibers and films can be easily rehydrated under humid conditions, e.g. at RH (relative humidity of air) above 50 %, and regain their adhesive properties; thus, they can be conveniently stored and handled in the dry state and converted *in situ* into the "active" adhesive form;
- hydrated fibers and films exhibit self-welding behavior, i.e. they can fuse with each other and enable the construction of more complex 2-dimensional and 3-dimensional structures and scaffolds;
- hydrated fibers and films exhibit strong adhesion properties with respect to a plurality of quite different materials and thus represent a class of very versatile, biocompatible and efficient adhesives.

These properties render the viscin fibers and films disclosed herein suitable for a broad range of applications, including biomedical applications.

The term "self-welding", as used herein, relates not just to a "sticking together" of fibers, but rather to an actual fusion of different fibers or parts thereof. In this process, one or more cycles of reversible hydration function as an eco-friendly and sustainable means for joining two fibers under ambient conditions.

Based on this humidity-initiated self-welding-behavior, it was possible to construct two-dimensional (and three-dimensional) objects from individual stiff mistletoe fibers, by simply arranging stiff pieces of mistletoe fiber in the dry state, exposing them to water vapor for a short time at the joints and then allowing them to air dry. The physical fusion of the fibers was confirmed using ESEM (data not shown). Indeed, the fibers lose their circular cross-section at the welding point and appear to merge together showing distortion in the microfibrillar texture on the surface of the fibers.

These observations confirm that the mistletoe viscin fibers are indeed self-welding using elevated moisture to join fibers at room temperature. Because the adhesive is integrated in the fiber, there is no need for additional glues, just water. This is a remarkable behavior for polymer fibers with a stiffness in the dry state of more than 14 GPa (i.e. exceeding that of Nylon by more than 3-fold).

Such 2D structures can be easily expanded into multilayer architectures similar to the additive manufacturing of 3D printed objects. For example, it is possible to prepare a multilayer 2D mesh in which multiple fibers laid over one another can envelop another layer of fibers oriented orthogonally, creating more complex, and presumably more stable junction points. Moreover, dried viscin fibers can be used to construct three-dimensional objects by a stepwise premanufacturing of several 2D mesh structures, which can later be assembled into the desired 3D shape and welded by local rehydration along the junction zones. And of course, it is also possible to build similar structures with freshly drawn self-adhesive viscin fibers.

Thus, a further aspect of the present invention relates to a method for preparing 2D and 3D viscin scaffolds comprising at least the following steps:
a) providing a multitude of dehydrated viscin fibers and/or viscin films in a non-adherent state,
b) rehydrating said viscin fibers and/or films, e.g. with water vapor or small volumes of liquid water, resulting in fibers and/or films with adhesive properties,
c) contacting and placing the rehydrated viscin fibers and/or films in a predetermined geometrical arrangement,
d) drying the fibers and/or films, preferably under ambient conditions, in particular air drying at a temperature in the range from 15°C to 30°C, preferably from 20°C to 25°C and at a relative humidity RH of less than 50%, resulting in a fusion of the viscin fibers and/or films in said predetermined geometrical arrangement and in the formation of a self-supporting 2D or 3D viscin scaffold in a predetermined shape.

The present invention also relates to self-supporting 2D and 3D viscin scaffolds obtained by self-welding, e.g. by the method as described above, which comprise viscin fibers and/or viscin films in a predetermined shape or geometrical arrangement and which exhibit adhesive properties in the hydrated state and are non-adherent under dry conditions.

Self-welding behavior under ambient conditions is not a typical property of most plastics, and fusion of two separate surfaces, would normally require bringing a thermoplastic to its melting point, and essentially remolding two surfaces into one. A notable exception is a class of supramolecular polymer elastomers known as vitrimers developed initially by Leibler and colleagues, which exhibit self-welding and self-healing behaviors based on reversible hydrogen bonding interactions (Cordier et al., 2008, Nature, 451: 977-80). However, this response requires the presence of unpaired hydrogen bond donors and acceptors on the surface, which decay on a freshly cut surface relatively quickly if two surfaces are not brought together immediately because new bonds are formed in the bulk rather than between two surfaces. In general, supramolecular polymers stabilized by reversible non-covalent interactions including H-bonding, metal coordination or ionic interactions will tend to be self-healing and possibly self-welding. However, these materials are typically extremely soft.

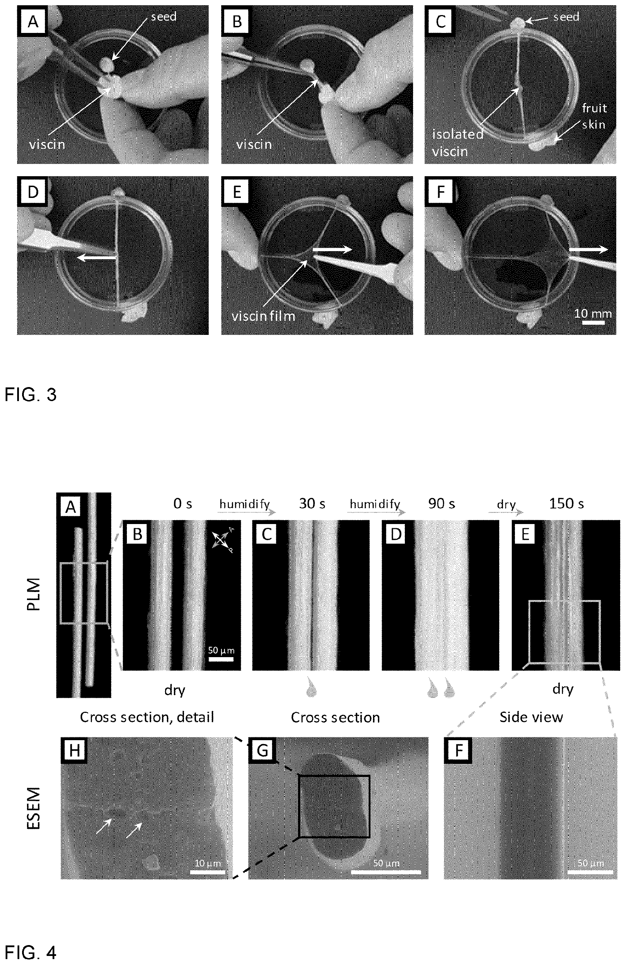

In contrast to typical supramolecular polymers, mistletoe viscin fibers are extremely stiff, yet flexible due to reinforcement with CMFs, and the self-welding response is triggered by simply cycling between low and high humidity conditions, resulting in the melding of multiple fibers (as shown in Fig. 4).

This combination of a water-responsive adhesive reinforced by stiff cellulosic fibrils provides excellent prospects for designing high-performance supramolecular composites, combining exceptional mechanical properties with intrinsic self-healing and self-welding response.

A further main aspect of the present invention relates to the use of mechanically isolated mistletoe viscin, e.g. obtainable by the method as described above, as a versatile adhesive for binding and gluing a plurality of materials.

A closely related aspect of the invention pertains to a method for joining or binding together a plurality of materials with diverse surface characteristics, wherein the materials are not plant tissues or plant-derived materials, comprising at least the following steps:
- contacting the surfaces of materials that are to be joined or bound together with a mechanically isolated viscin, e.g. obtained by the method as described above, or a viscin film as defined above, wherein the viscin is in the hydrated/wet and adhesive state,
- optionally applying pressure to ensure a close contact of all the materials,
- drying the resulting composite material.

Surprisingly, it has been found that mechanically isolated mistletoe viscin (and a film or other structure prepared therefrom) exhibits excellent adhesion properties not only with respect to plant tissues or plant-derived materials (as observed with mistletoe seeds digested and excreted by birds in nature) but rather with respect to materials with quite diverse surface characteristics, including surfaces which may present both polar and/or nonpolar moieties.

More specifically, these materials may be selected from the group comprising synthetic polymeric materials or plastics, including but not limited to polyethylene, polycarbonate, polytetrafluorethylene, and inorganic materials, including but not limited to metals and metal alloys such as bronze, aluminum and steel, glass, ceramics, mica.

Also, these materials may be selected from the group comprising animal tissue, including but not limited to mammalian skin and cartilage.

These favorable adhesive properties are also exhibited by the viscin films and/or 2D or 3D viscin scaffolds when in a wet or (re)hydrated state.

Consequently, a closely related aspect of the present invention pertains to an adhesive comprising viscin films and/or 2D or 3D viscin scaffolds, in particular an adhesive tape, which is capable to provide adhesion on and between a plurality of materials with diverse surface characteristics, including surfaces which may present both polar and/or nonpolar moieties.

In a more specific embodiment these materials are selected from the group comprising synthetic polymeric materials or plastics, including but not limited to polyethylene, polycarbonate, polytetrafluorethylene, and inorganic materials, including but not limited to metals and metal alloys such as bronze, aluminum and steel, as well as glass, ceramic, mica.

In another specific embodiment these materials are selected from the group comprising animal tissue, including mammalian skin and cartilage. The observation that mistletoe viscin can adhere to cartilage is especially remarkable considering the enormous challenges in developing effective adhesives for wet surfaces in biological tissues (see, e.g. Li et al., 2017, Science, 357: 378-81).

These multimaterial adhesive properties of viscin are rather surprising, since no single chemical or mechanical adhesion mechanism is able to provide strong adhesion to both hydrophilic (e.g. mica, biological tissues) and hydrophobic (e.g. PTFE) surfaces. In particular, viscin also binds to freshly cleaved mica, on which surface roughness is minimal, and to cartilage, which most adhesives functioning purely based on mechanical mechanisms cannot adhere to. It appears that the adhesion properties of viscin are chemically highly versatile.

A further related aspect of the present invention, therefore, pertains to wound sealant and coating compositions, in particular for sealing and/or covering wounds in mammalian tissue including but not limited to skin, comprising mechanically isolated mistletoe viscin in the hydrated/wet state. In one preferred embodiment, these compositions comprise further components such as one or more plant oils, in particular selected from the group comprising walnut oil, olive oil, rapeseed oil, linseed oil, and optionally further additives.

With respect to skin coatings and wound sealants, the processability of viscin into a coating is incredibly facile. No further additives are required - as long as the viscin stays hydrated, the coatings can be formed and applied under ambient conditions. Indeed, the humidity from skin moisture appear to be enough to keep the viscin film pliable and sticky for at least several days. The mechanical integrity of the coatings is easily demonstrated and arises presumably from the presence of cellulose filaments and microfibrils within the films. The coatings remain firmly attached during brief washing in water (e.g. hand washing); yet, are easily removable with a bit of friction, providing the ideal properties for a tissue sealant.

Moreover, mixing the viscin with plant oils results in a larger, less sticky, smooth film that is comfortable to "wear" and more flexible. This means that there are no restrictions in using coated hands besides ensuring not to soak the film for too long in water since it completely rehydrates and swells dramatically, and will wash it off at some point.

As opposed to currently available petroleum-based or nitrocellulose-based wound sealants, the viscin films are sustainable natural products with no synthetic additives, are environmentally friendly, are apparently biocompatible (no skin irritations were observed), are biodegradable and even function under wet conditions (suggesting these would still work in the presence of bodily fluids such as blood).

In an alternative embodiment, a wound sealant is obtained by using a preformed dried viscin film which can be rehydrated *in situ* and applied on the wound instead of the native isolated viscin tissue above.

This embodiment is particularly advantageous in that it allows to use a dried viscin film which can be easily stored (e.g. as a component of a first-aid kit) and handled and which - after rehydration- readily adapts to various surface shapes and surface topographies, e.g. curved or flat, rough or smooth surfaces, and can be easily and fast applied to an extended wound area.

Consequently, the present invention further encompasses a dried viscin film for use, after rehydration under humid conditions, as a wound sealant or a medical kit comprising said dried viscin film.

### Brief Description of the Figures

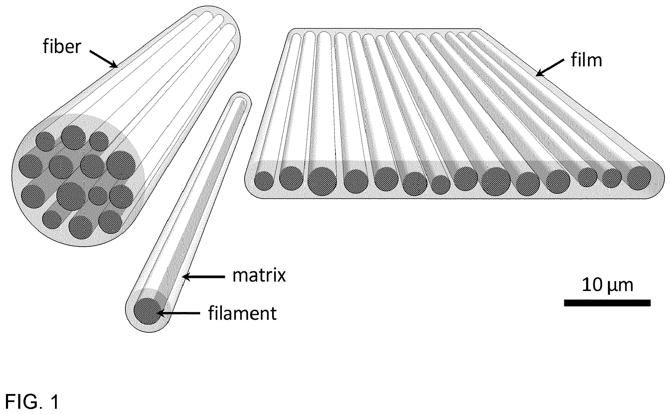

**Fig. 1** shows schematically the structural relationship between viscin filaments (extended cells) and corresponding fibers and films.

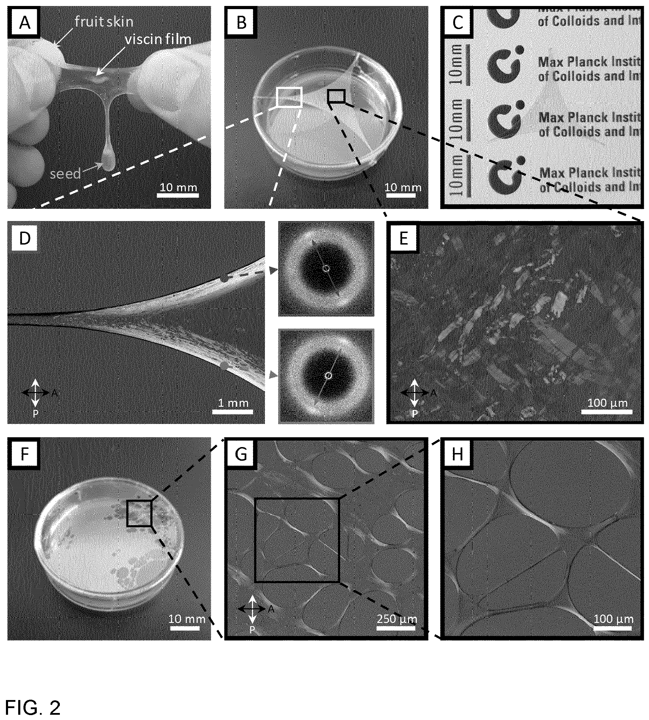

**Fig. 2** shows the manual preparation of a self-supporting viscin film attached to a Petri dish. A) Removing of the seed from the berry; B) Pulling the viscin out of the berry and depositing the same in the center of a Petri dish bottom; C) Attaching the seed to one edge of the Petri dish, and the remaining fruit skin to an opposite edge; D) Pulling the viscin strand towards the left edge of the Petri dish; E) Attaching the viscin to a third connection point, which leads to the formation a triangular shaped viscin film, and pulling an edge of the freshlyformed film towards the right side of the Petri dish; F) A quadrangular viscin film is being formed.
**Fig. 3** shows structural and optical properties of viscin films. A) Image of a freshly formed and flexible viscin film; B) Free standing adhesive viscin film drawn into a triangular shape and glued to the edge of a Petri dish; C) A dried viscin film is dimensionally stable and highly transparent; D) PLM image showing a detail of (B) revealing the cellulose orientation along the contours of a film, cellulose orientation was also confirmed with wide-angle X-ray diffraction shown in the adjacent diffraction pattern from 2 selected points along the film contour; E) PLM image of the film center from (B); F) A viscin film which locally collapsed into a porous structure; G) PLM image showing a detail of a porous region from (F); H) Detail from (G) showing that the film locally collapsed into small fibers.
**Fig. 4** shows the self-welding of viscin fibers. A) PLM image of 2 dried viscin fibers; B) Detail of (A); C) Slightly swollen fibers after 30 s of exposure to saturated water vapor (RH ∼ 100%); D) Fused fibers at maximum swelling after 90 s of rehydration; E) 60 s after maximum swelling; F) ESEM image showing a side view of the fused fibers; G) ESEM image of a fused fiber cross section; H) Detail of (G) revealing that the interface between the fused fibers disappears.

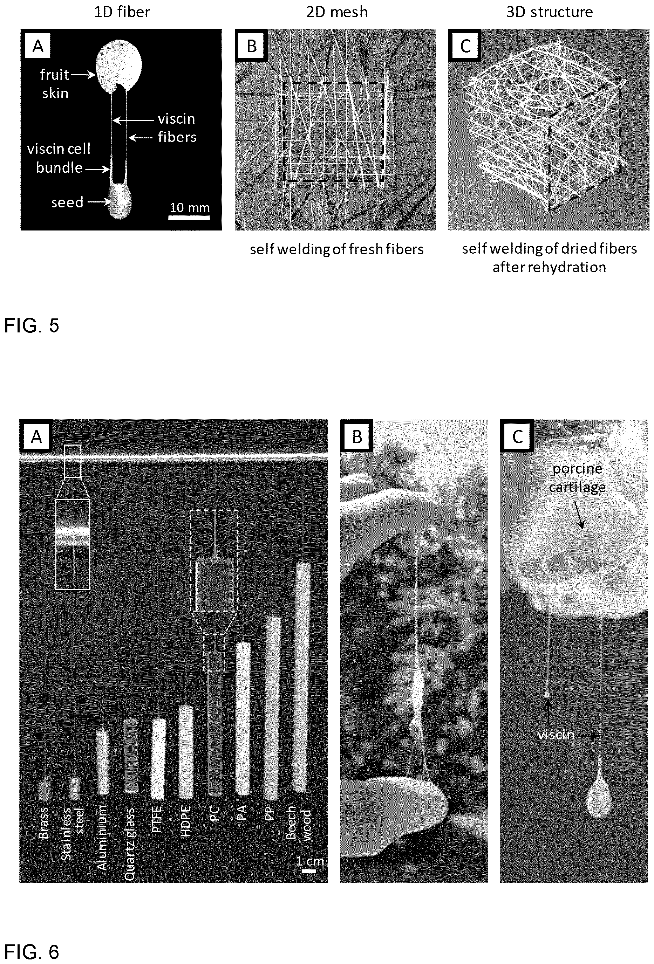

**Fig. 5** shows various structures made from self-welding viscin fibers. A) Manually drawn viscin fibers; B) A two-dimensional mesh created from self-welding viscin fibers; C) A hollow cube formed of premanufactured two-dimensional meshes welded by rehydration.
**Fig. 6** shows the multimaterial adhesive properties of viscin. A) Cylinders from 10 selected materials with different surface chemistries are supported by a viscin fiber each attached to the top surface of the cylinder and a laboratory stand; B) A viscin fiber adhered between two fingers supporting the seed; C) *V. album* seeds adhering to porcine cartilage.

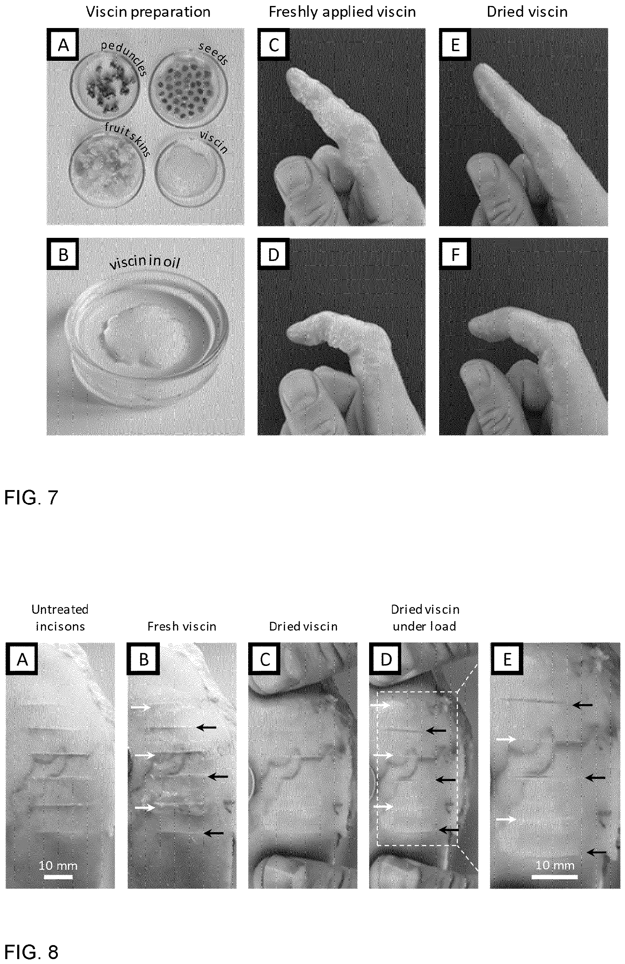

**Fig. 7** shows the preparation and application of viscin bandages. A) Mechanically separated fractions of *V. album* berries: peduncles, seeds, skins and flesh and viscin; B) Viscin submerged in walnut oil; C) translucent, milky viscin coating from (B) freshly applied to human skin, covering a finger; D) the freshly applied viscin allows free movement of the covered finger; E) the viscin coating dried into a thin transparent film; F) the dried flexible viscin coating still allows free movement of the finger.
**Figure 8** shows an image series of an artificial wound treatment with viscin. A) Parallel cuts were made with a razor blade on a porcine skin; B) Three incisions were selected as reference (black arrows) while the remaining incisions were used for viscin treatment (white arrows), freshly isolated viscin was spread over the selected incisions; C) Applied viscin after drying; D) Dried viscin sealant loaded perpendicular to the incisions; E) Detail from (D) showing the opened reference incisions under load while the dried viscin sealant keeps the incisions sealed and closed.

The following Examples are provided to illustrate the present invention in more detail, however, without limiting the same to the specific conditions and parameters thereof.

### EXAMPLE 1

### Preparation of self-supporting viscin films

### A. Manual drawing of dimensionally stable, free standing 2D films:

Single berries were placed into a small glass Petri dish with a diameter of 5 cm. A large incision was made on the top of the berry with a razor blade. Through this opening in the fruit skin und the fleshy layer underneath the seed was carefully taken out of the berry with fine tweezers and put aside in the Petri dish close to the remaining berry (Fig. 2-A).

Then the viscin was carefully isolated by hand using tweezers with a broad tip in a stepwise procedure as follows: The tweezers were used to grab the viscin cell clusters, located on the inside of the fruit flesh, and pull it out of the berry with a short and slow movement to reduce the mechanically induced formation of fibers as best as possible. The viscin was deposited on the bottom of the Petri dish next to the seed. The last two steps were repeated until the viscin inside the berry was depleted (Fig. 2-B). The remains of the fruit skin were grabbed with tweezers and glued to the edge of the Petri dish exploiting the natural adhesive properties of the viscin. Then the seed was glued to the opposite side of the Petri dish and the isolated viscin was carefully lifted off the bottom, which leads to the formation of a thick viscin strand between the seed and the fruit skin (Fig. 2-C). To form a triangular shaped film, one can grab the strand at any location and pull it towards an arbitrary edge of the Petri dish where it can be fixed by pressing the newly formed pointed end of the film onto the glass for ∼30 s (Fig. 2-D+E). While pressing the film has to be gently kept under tension to maintain the film shape because otherwise the hydrated viscin film promptly collapses into three thick strands. To form a quadrangular shape one can grab another film edge and pull it towards any edge of the Petri dish and fix it (Fig. 2-E+F).

A quick repetition of this procedure allows to form various 2-dimensional polygons as long as the viscin is hydrated. In order to achieve more complex 3-dimensional film geometries one can select different height levels for the exterior connection points. The resulting film spans the area between the connecting points like a stretched tarpaulin. The viscin films quickly dry under ambient conditions. The thinner center area of the films dries faster than the thicker edges. After drying the dimensionally stable films can be manipulated with tweezers and cut into any shape with tools such as razor blades, knifes or scissors. Tapered ends can also be simply sheared off with a blunt tool.

**B.** Based on these manual methods for films formation and the high reproducibility, it is possible to design a system for automating films formation utilizing a multiaxial tensile tester, for instance. The use of such a system allows quantitatively controlling the directions and rates of strain, which in turn enables or facilitates to obtain a desired structure and desired properties of the resulting films.

A freshly formed (or rehydrated) adhesive viscin film can be applied onto a wide range of material surface and readily adapts to various surface shapes and surface topographies, e.g. curved or flat, rough or smooth surfaces.

### EXAMPLE 2

### Characterization of viscin films

Fig. 3 illustrates the structural and optical properties of viscin films prepared as described in Example 1: A) Image of a freshly formed and flexible viscin film drawn from a compressed berry of *V. album.* Scale bar: 10 mm; B) Free standing viscin film drawn into a triangular shape and glued to the edge of a Petri dish making use of the natural adhesive properties. Scale bar: 10 mm; C) A dried viscin film is dimensionally stable and highly transparent; D) polarized light microscopy (PLM) image showing a detail of (B) revealing the cellulose orientation along the contours of a film. Scale bar 1 mm. Cellulose orientation was also confirmed with wide-angle X-ray diffraction (WAXS) shown in the adjacent diffraction pattern from 2 selected points along the film contour. The arrows in the diffraction patterns mark the distinct equatorial cellulose diffraction spots. E) PLM image of the film center from (B) where viscin cells are found to be randomly oriented and mostly unstretched. Scale bar: 250 µm. F) A viscin film which locally collapsed into a porous structure. Scale bar: 10 mm. G) PLM image showing a detail of a porous region from (F). Scale bar: 250 µm. H) Detail from (G) showing that the film locally collapsed into small fibers. The cellulose is highly aligned along the fiber directions and the pore contours as indicated by the polarization colors.

As evident from Fig. 3, the transparent film stiffens when dried, retaining its integrity and shape (Fig. 3-C). PLM imaging of the film reveals orientation of the cellulose microfibrils along the local contours of the anchoring points (Fig. 3D). This orientation was verified with wide-angle X-ray scattering, showing that cellulose is aligned along the apparent stress fields. In the middle of the film, in contrast, numerous randomly oriented unstretched viscin cells could be observed (Fig. 3-E) - which could ostensibly supply further extensibility of the film in the wet state. However, a viscin film, which is excessively stretched will fail at some point. The failure is not characterized by a total rupture of the film but is rather initiated by a local collapse of the film into a porous architecture (Fig. 3-F). The arising pores are connected via thin highly oriented fiber segments ensuring the mechanical integrity of the remaining film (Fig. 3-G+H). As revealed by PLM, the cellulose orientation follows the local contours of the pores.

### EXAMPLE 3

### Self-welding properties of viscin fibers

Fig. 4 demonstrates the self-welding of viscin fibers: A) PLM (polarized light microscopy) image of 2 loose ends of dried viscin fibers oriented at 45° to the polarization filters; B) Detail of (A). Scale bar: 50 µm; C) Fibers were brought into contact. Slightly swollen fibers after 30 s of exposure to saturated water vapor (RH ∼ 100%); D) Fibers at maximum swelling after 90 s of rehydration; E) 60 s after maximum swelling (total experimental time of 150 s), the diameter of the fused fibers is reduced dramatically again; F) ESEM image showing a side view of the fused fibers. Scale bar: 50 µm. G) ESEM image of a fused fiber cross section. Scale bar: 50 µm; H) Detail of (G) revealing that the interface between the fused fibers gets lost. Arrows: voids due to possible inclusions at the former fiber-fiber interface. Scale bar: 10 µm.

As evident from Fig. 4, fibers do not interact adhesively in the dry state when they are brought into contact. However, by raising the RH to above ∼40%, it could be observed that the fibers swell slightly and when brought into contact begin to adhere and deform at the interface (Fig. 4-C). Further raising the humidity by exposing the fiber ends to saturated water vapor (∼100% RH) leads to an intense swelling of the fibers where the fibers further deform along the interface between the fibers, which gradually disappears (Fig. 4-D). Cutting off the water vapor leads to a sudden drop of the RH back down below 40%, after which it was observed that the fibers become physically fused to one another (Fig. 4-E+F). The fused fiber ends were cut and the cross-section was investigated with environmental scanning electron microscopy (ESEM), confirming that the interface between the formerly separate fibers is largely lost (Fig. 4-G+H).

Based on this humidity-initiated self-adhesion between viscin fibers and subsequent self-welding, it was possible to construct two-dimensional (and three-dimensional) objects from individual stiff mistletoe fibers, by simply arranging stiff pieces of mistletoe fiber in the dry state, exposing them to water vapor for a short time at the joints and then allowing them to air dry. The physical fusion of the fibers was confirmed using ESEM (data not shown). Indeed, the fibers lose their circular cross-section at the welding point and appear to merge together showing distortion in the microfibrillar texture on the surface of the fibers. These observations confirm that the mistletoe viscin fibers are indeed self-welding using elevated moisture as a soldering agent at room temperature.

Such 2D structures can be easily expanded into multilayer architectures similar to the additive manufacturing of 3D printed objects. For example, it is possible to prepare a multilayer 2D mesh in which multiple fibers laid over one another can envelop another layer of fibers oriented orthogonally, creating more complex, and presumably more stable junction points. Moreover, dried viscin fibers can be used to construct three-dimensional objects by a stepwise premanufacturing of several 2D mesh structures, which can later be assembled into the desired 3D shape and welded by local rehydration along the junction zones. And of course, it also possible to build similar structures with freshly drawn self-adhesive viscin fibers.

Fig. 5 shows various structures made from drawn viscin fibers. A) Manually drawn viscin fibers; B) A two-dimensional mesh created from self-welding viscin fibers. After drying the mesh can be cut from the cardboard frame (dotted line: cutting contour); C) A hollow cube formed of premanufactured two-dimensional meshes welded by rehydration (dotted line: outlines of an individual 2D mesh from (B)). Scale bars: 10 mm.

### EXAMPLE 4

### Multimaterial adhesive properties of viscin

Viscin was isolated from single berries as described for the making of viscin films above where the seed and the fruit skin were cut off at both ends of the isolated viscin. The viscin of a single berry was deposited on the top surface of an erected cylinder and drawn into a fiber which was attached to a horizontal steel bar of a laboratory stand placed above the cylinder. This way 10 viscin fibers were made connecting the metal bar with a 10 lined up cylinders each consisting of a different material: brass, aluminum, stainless steel, quartz glass, polytetrafluoroethylene (PTFE), high density polyethylene (HDPE), polycarbonate (PC), polyamide (PA), polypropylene (PP) and Beech wood. Each cylinder had a diameter of 10 mm and a weight of ∼10 g. The viscin was allowed to dry for 2 h until the metal bar was lifted by 10 cm so the dried viscin fibers were carrying the load of the free hanging cylinders.

To test the adhesive properties of viscin in combination with cartilage a pork knee joint was used from a fresh pork leg, bought from Fleischerei Domke, Berlin. The cartilage from the knee joint was isolated from the surrounding tissue. Viscin and seed were extracted as described for the making of viscin films and attached to the freshly exposed cartilage tissue. Fig. 6 demonstrates the multimaterial adhesive properties of viscin: A) Cylinders from 10 selected materials with different surface chemistries are supported by a viscin fiber each attached to the top surface of the cylinder and a laboratory stand. Cylinder diameter: 1 cm, cylinder weight: 10 g. Scale bar: 1 cm; B) A viscin fiber adhered between two fingers supporting the seed; C) Two *V. album* seeds adhering to porcine cartilage. One seed is directly attached to the cartilage via the hydrated viscin layer surrounding the seed. The other seed is connected via a freshlyformed viscin fiber.

### EXAMPLE 5

### Preparation and application of viscin bandages and wound sealants

The viscin of multiple individual berries can be mixed. Therefore, the viscin can be isolated and collected in a Petri dish as described in the previous section after which the isolated material can be cut free from the seed on one end and from the fruit flesh and skin on the other end using a razor blade or scissors. The viscin of a second berry can simply be added to the existing viscin. The two viscin units instantly adhere and can only be separated again within the first minute after initial contact. Afterwards they permanently fuse into a sticky mass. By adding viscin of more berries the total mass of viscin can be increased at will. The mixing can be enhanced by slowly stirring the viscin mass with a spatula. The sticky coherent mass can be picked up from the Petri dish either by hand or with tools like tweezers or spatulas. The bulk viscin can then be deposited onto the skin region it is supposed to seal. Starting from the initially covered region the viscin can be readily distributed by shearing the material along its edges where it instantly adheres to the newly covered area. Within several minutes the viscin dries into a transparent film. If the viscin mass is not sufficient enough to cover the desired surface, the remaining area can simply be covered by adding isolated viscin from more berries. This can be done with freshly applied viscin but also when the viscin sealing already partly or even completely dried. Alternatively, the freshly mixed viscin can be manually drawn into films as described above for single berries which can then directly be applied onto the skin. Dried films can readily be peeled or rubbed off of the skin again, leaving no visible traces.

In another embodiment, native viscin was isolated and mixed as described above and then submerged in commercially available olive oil (de Cecco, Italy) or walnut oil (Kunella Feinkost, Germany) for 5 minutes including gentle stirring with a spatula. Afterwards the coherent viscin mass was removed from the oil bath and the oil covered viscin is kneaded by hand for one minute. The resulting viscin-oil mixture was applied to human skin analog to the oil free viscin as described above. The applied viscin was allowed to dry into a transparent film within a few minutes. After testing the film can readily be removed off the skin by peeling or rubbing.

The oil-treated viscin was not as sticky as the native viscin, allowed a much easier processing, but still revealed a notable adhesion to human skin. The oil-treated viscin exhibited only a weak tendency to form fibers combined with an improved coherence. The oil-processed viscin feels smooth and silky and can be kneaded like a dough, stretched into stable films and applied to the skin where it can be further redistributed over the designated area (Fig. 7-C+D). Within only a few minutes even thickly applied viscin dries into a smooth transparent coating (Fig. 7-E). In contrast to the native viscin, the oil-treated viscin does not show any perceptible tack and allows one to grab or touch things without getting stuck to the surface and is so flexible that is does not restrict any movements (Fig. 7-F). Indeed, it is not perceptible on the skin. Similar to the native viscin, defects can be repaired by adding further material (additive manufacturing) or by locally rehydrating the coating which enables further manipulation, consistent with the self-welding nature of the material. To wrap an entire finger with a viscin coating as presented in Figure 7C+D it requires the viscin of about 10-15 berries.

To investigate the potential of viscin as a wound sealant, incisions were made in porcine skin (non-living) using a razor blade (Fig. 8-A). The incisions were sealed by spreading native isolated viscin tissue over the cut (Fig. 8-B), and allowing it to dry (Fig. (8-C). The silky, glossy viscin sealant remained attached upon drying and even when a load was applied to the skin, the cut remained sealed, while nearby unsealed incisions opened easily (Fig. 8-D+E). Tested on live human skin (no incisions inflicted!), such a viscin sealant remained attached for a period of more than 3 days. The sealant always retained a minor tack on its surface. Aside from that, it remained flexible, allowing free movement when performing everyday tasks like using tools, cutlery or riding a bike and was even resistant to short time rinsing with water, e.g. when washing the skin under running water. To remove the seal, friction could be used by simply rubbing the sealed area.

In an alternative embodiment, a preformed and rehydrated viscin film is applied on the wound instead of the native isolated viscin tissue above.

This embodiment is particularly advantageous in that it allows to use a dried viscin film which can be easily stored and handled and which -after rehydration- readily adapts to various surface shapes and surface topographies, e.g. curved or flat, rough or smooth surfaces, and can be easily and fast applied to an extended wound area.

## Claims

1. A self-supporting 2D mistletoe viscin film comprising a 2-dimensional multi-axial oriented array of viscin cellulose filaments within a matrix, wherein viscin, as used herein, refers to the mucilaginous tissue surrounding the pseudoseeds of mistletoe plants, the tissue comprising viscin cells, which cells exhibit a characteristic cell wall structure of coiled cellulose fibrils and are organized in clusters or bundles in a natural humidity-responsive adhesive matrix.

2. The self-supporting viscin film according to claim 1 having locally oriented cellulose filaments embedded within a humidity-responsive matrix which film exhibits adhesive properties in the hydrated/wet state and which film is non-adherent and transparent in the dehydrated/dry state.

3. A method for the mechanical isolation of viscin comprising at least the following steps:
a) opening the pseudoberries of mistletoe plants by mechanical means, e.g. applying compression forces to the pseudoberries or cutting the pseudoberries,
b) extracting the seeds,
c) separating the viscin tissue attached to the seeds and the pseudoberry skins from said seeds and pseudoberry skins,
d) optionally further processing or purifying of the viscin tissue obtained in step c).

4. A method for preparing a self-supporting 2D viscin film according to any one of claims 1-2 comprising at least the following steps:
a) providing mechanically isolated viscin tissue,
b) applying a multi-axial tensile load onto said viscin tissue, resulting in the formation of a viscin film comprising a 2-dimensional array of viscin cellulose filaments oriented locally along the direction of the tensile load during formation,
c) drying the resulting film,
d) optionally further processing after rehydration.

5. The method according to claim 4, wherein step b) comprises
i) forming hydrated viscin tissue into one or more self-supporting strands and anchoring the same in a predetermined distance at two elevated points of a substrate by means of the natural adhesion of the viscin and without additional contact with the substrate,
ii) subsequently drawing the still hydrated viscin strand along at least one other axis forming a 2D film with at least three anchor points.

6. A method for preparing 2D and 3D viscin scaffolds comprising at least the following steps:
a) providing a multitude of dehydrated viscin fibers and/or viscin films in a non-adherent state,
b) rehydrating said viscin fibers and/or films, resulting in fibers and/or films with adhesive properties,
c) contacting and placing the rehydrated viscin fibers and/or films in a predetermined geometrical arrangement,
d) drying the fibers and/or films, resulting in a fusion of the viscin fibers and/or films in said predetermined geometrical arrangement and in the formation of a self-supporting 2D or 3D viscin scaffold in a predetermined shape.

7. Self-supporting 2D and 3D viscin scaffolds, e.g. obtainable by the method of claim 6, which comprise viscin fibers and/or viscin films in a predetermined shape or geometrical arrangement and which exhibit adhesive properties in the hydrated state and are non-adherent under dry conditions.

8. Use of mechanically isolated mistletoe viscin, e.g. obtainable by the method according to claim 3, as a versatile adhesive for binding and gluing a plurality of materials with diverse surface characteristics, including surfaces which may present both polar and/or nonpolar moieties, wherein the materials are not plant tissues or plant-derived materials.

9. A method for joining or binding together a plurality of materials with diverse surface characteristics, wherein the materials are not plant tissues or plant-derived materials, comprising at least the following steps:
- contacting the surfaces of materials that are to be joined or bound together with a mechanically isolated viscin or a viscin film as defined in claims 1-2 above, which is in the hydrated and adhesive state,
- optionally applying pressure to ensure a close contact of all the materials,
- drying the resulting composite material.

10. The use according to claim 8 or the method according to claim 9, wherein the materials are selected from the group comprising synthetic polymeric materials or plastics, including but not limited to polyethylene, polycarbonate, polytetrafluorethylene, and inorganic materials, including but not limited to metals and metal alloys such as bronze, aluminum and steel, glass, ceramic, mica.

11. The use according to claim 8 or the method according to claim 9, wherein the materials are selected from the group comprising animal tissue, including but not limited to mammalian skin and cartilage.

12. An adhesive comprising viscin films and/or 2D or 3D viscin scaffolds, in particular an adhesive tape, which is capable to provide adhesion on and between a plurality of materials with diverse surface characteristics, including surfaces which may present both polar and/or nonpolar moieties.

13. The adhesive according to claim 12, wherein the materials are selected from the group comprising synthetic polymeric materials or plastics, including but not limited to polyethylene, polycarbonate, polytetrafluorethylene, and inorganic materials, including but not limited to metals and metal alloys such as bronze, aluminum and steel, glass, ceramic, mica.

14. The adhesive according to claim 13, wherein the materials are selected from the group comprising animal tissue, including mammalian skin and cartilage.

15. A wound sealant and coating, in particular for sealing and/or covering wounds in mammalian tissue including but not limited to skin, comprising mechanically isolated mistletoe viscin in the hydrated/wet state, a plant oil, in particular selected from the group comprising walnut oil, olive oil, rapeseed oil, linseed oil, and optionally further additives.

16. A dried viscin film for use, after rehydration under humid conditions, as a wound sealant or a medical kit comprising said dried viscin film.

17. The viscin film according to claims 1-2 and 16 or the viscin scaffolds according to claim 7, the methods according to claims 3-6 or 9-11, the uses according to claims 8 and 10-11, the adhesive according to claims 12-14, or the wound sealant or medical kit according to claims 15-16, wherein the viscin is a misteltoe viscin derived from pseudoberries of mistletoe plants selected from the group comprising the European mistletoe, i.e. *Viscum album* L., and related species or subspecies, in particular Viscum minimum, Viscum cruciatum, Viscum album L. coloratum, Viscum album var. rubro-aurantiacum Makino, Viscum album L. var. lutescens Makino, and Phthirusa pyrifolia.
